# EUROPEAN PATENT SPECIFICATION

(11) **EP 3 153 577 B1**
(45) Date of publication and mention of the grant of the patent: **14.11.2018**
(21) Application number: 16191784.4
(22) Date of filing: 30.09.2016
(51) Int. Cl.: C12N 7/00, A61K 35/76, A61K 39/17

(54) **METHOD FOR MODIFICATION OF HEAT RESISTANCE OF NEWCASTLE DISEASE VIRUS AND USE THEREOF**
VERFAHREN ZUR ÄNDERUNG DER HITZEBESTÄNDIGKEIT DES NEWCASTLE DISEASE VIRUS UND DESSEN VERWENDUNG
PROCÉDÉ DE MODIFICATION DE LA RÉSISTANCE À LA CHALEUR DU VIRUS DE LA MALADIE DE NEWCASTLE ET SON UTILISATION

(30) Priority: 09.10.2015 CN 201510647982
(43) Date of publication of application: 12.04.2017
(73) Proprietor: Institute of Animal Husbandry and Veterinary Science, Hubei Academy of Agricultural Sciences, Wuhan City, Hubei 430072 (CN)
(72) Inventor: SHAO, Huabin, Wuhan City Hubei 430072 (CN); YU, Qingzhong, Wuhan City Hubei 430072 (CN); WEN, Guoyuan, Wuhan City Hubei 430072 (CN); LUO, Qingping, Wuhan City Hubei 430072 (CN); WANG, Honglin, Wuhan City Hubei 430072 (CN); ZHANG, Rongrong, Wuhan City Hubei 430072 (CN); AI, Diyun, Wuhan City Hubei 430072 (CN); ZHANG, Tengfei, Wuhan City Hubei 430072 (CN); LUO, Ling, Wuhan City Hubei 430072 (CN); WANG, Hongcai, Wuhan City Hubei 430072 (CN)
(74) Representative: Laufhütte, Dieter

(56) References cited:
- KR-A- 20150 026 818
- US-A1- 2014 287 496
- WEN G ET AL: "Molecular basis for the thermostability of Newcastle disease virus", SCIENTIFIC REPORTS, vol. 6, 3 March 2016 (2016-03-03), page 22492, XP055345731, DOI: 10.1038/srep22492

## Description

### Technical Field

The present disclosure relates to reverse genetic manipulation of viruses, and in particular, to a method for modification of heat resistance of Newcastle disease virus and application thereof. More specifically, the present disclosure relates to a method for modification of heat resistance of a non-heat-resistant Newcastle disease virus, a heat resistance-modified recombinant Newcastle disease virus and a heat-resistant live vaccine comprising the virus.

### Technical Background

Newcastle disease (ND), caused by Newcastle disease virus (NDV), is a contagious and devastating disease of poultry that can result in high mortality. ND is defined by the International Office of Epizootics as one of the two List A poultry diseases, the other being avian influenza. NDV belongs to the genus Avulavirus in the Paramyxoviridae family. The genome of NDV is a single-stranded, negative-sense, and non-segmental RNA, with a full-length of 15,186 nucleotides (nt) conforming to the "rule of six", and containing 3' leader sequence of 55-nt and 5' trailer sequence of 55-nt between which there are 6 structural genes, which are: 3'-NP (nucleocapsid protein)-P (phosphorylated protein)-M (matrix protein) -F (fusion protein)-HN (hemagglutinin-neuraminidase protein) -L (large polymerase protein)-5' in sequence. At least 7 proteins are encoded in the genome. Each of the structural genes comprises, at the beginning and end of the open reading frame (ORF) thereof, a start sequence and an end sequence, respectively, which serve as transcription regulatory sequences. Among each of these genes, there is/are 1 to 47 intergenic spacers (IGS).

Since its first breakout in Java, Indonesia, and in Newcastle, England, in 1926, ND in chickens has been prevalent throughout the world except in Oceania, and has resulted in great economic loss globally. As one of the most devastating poultry diseases in China, ND has been prevalent in many regions in China. ND is characterized by breathing difficulty, diarrhea, nervous disorder, wide-range mucosal bleeding in alimentary tract, and so on. ND is mainly controlled through immunprofilaxis, and is an epidemic disease against which compulsory immunization is provided by the state. In recent years, with the wide use of vaccines, wide-scale breakout and prevalence of ND has been greatly prevented. However, ND starts to have new epidemic characteristics, such as emergence of atypical NDs, mixed infection, and enlargement of host range.

Currently, ND vaccines internationally produced and used are classified into two types, namely live vaccines and inactivated vaccines. The live vaccines include low virulent strain vaccines and moderate virulent strain vaccines. The low virulent strain vaccines include lineage II vaccines (B1), lineage III vaccine (LaSota strain), clone 30, V4, etc., and the moderate virulent strain vaccines include lineage I vaccines, Roskin strain, Komorov strain, Hert 33 strain, Mukteswar strain, etc. Some low virulent live vaccines have a unique heat-resistant characteristic and are known as heat-resistant live vaccines, representative strains thereof including V4, HB92, TS09-C strains, etc. This type of vaccines has advantages of being heat-resistant, low virulent, highly effective in immunization, infectious within the group, and capable of being administered in multiple approaches (e.g., food mixing and spraying), and is applicable to the prevention and control of Newcastle disease in a variety of birds such as chicken, pigeons, and quails. Compared with other non-heat-resistant vaccines, these heat-resistant live vaccines offer more advantages in southern areas with generally high temperature and rural areas with poor cold chain conditions, and play an important role in preventing and controlling of NDs. However, at present, the most widely used low virulent strain vaccine LaSota strain and moderate virulent strain are all non-heat-resistant NDV strains. Therefore, it is of great significance to discuss the heat resistance mechanism of NDV and discuss how to improve the heat resistance of other NDV strains such as LaSota strain.

Among numerous literature documents about reverse genetic manipulation of NDV, no report is found on modification of heat resistance of NDV. For example, Chinese patent application No. 200510097997.8, entitled "Newcastle disease LaSota vaccine strain reverse genetic manipulation system and use thereof', discloses a non-heat-resistant live vaccine vector based on an NDV LaSota vaccine strain, but the application does not involve any method for modification of heat resistance of NDV. Chinese patent application No. 200610075781.6, entitled "Recombinant attenuated Newcastle disease LaSota vaccine strain expressing HA protein of Avian Influenza Virus H5 subtype", discloses an avian influenza-ND bivalent genetic engineering live vaccine constructed by using an ND LaSota vaccine strain vector, but the application does not involve any method for modification of heat resistance of NDV. Chinese patent application No. 201310090099.4, entitled "Newcastle disease virus heat resistant live vaccine vector system and application thereof', discloses a vector system of NDV heat-resistant live vaccines, but the application does not involve any method for modification of heat resistance of NDV. Korean patent application No. 20150026818 A discloses a chimeric NDV strain BP ADNDM with improved heat resistance. Said strain is obtained by reverse genetics and comprises the NP, P, M and L genes of a mildly heat resistant NDV and the F and HN genes of a pathogenic NDV strain.

### Summary of the Invention

The objective of the present disclosure is to provide a method for modification of heat resistance of NDV and use thereof, so as to eliminate the shortcomings of the existing technologies.

In order to achieve the above objective, the present disclosure provides the following technical solutions.

Provided is a method for modification of heat resistance of NDV. The method comprises steps of:
a. constructing a transcription plasmid of a non-heat-resistant NDV;
b. replacing the hemagglutinin-neuraminidase (HN) gene in the transcription plasmid of the non-heat-resistant NDV with the HN gene of a heat-resistant NDV, so as to obtain a modified transcription plasmid; and
c. co-transfecting a host cell with the modified transcription plasmid and three other helper plasmids to, so as to obtain a heat resistance-modified recombinant NDV,
wherein the heat-resistant Newcastle disease virus is the TS09-C strain with the HN gene having the sequence of SEQ ID NO: 1, and
the three helper plasmids are capable of expressing nucleoprotein (NP), phosphoprotein (P), and large polymerase protein (L) of NDV, respectively.

In one embodiment, the transcription plasmid of the non-heat-resistant NDV expresses the whole genomic cDNA of the non-heat-resistant Newcastle disease virus.

In one embodiment, the non-heat-resistant Newcastle disease virus is the LaSota strain.

The invention further relates to a heat resistance-modified recombinant NDV, which is the rT-HN strain deposited in China Center for Type Culture Collection since August 3, 2015, in Wuhan University, Wuhan, China, under Accession Number CCTCC NO: V201529.

Still further, the invention relates to a heat-resistant live vaccine for preventing and/or controlling Newcastle disease comprising said recombinant NDV rT-HN strain.

Technical principles of the present disclosure are described as follows.
1) The NP, P, M, F, HN, and L genes of a heat-resistant TS09-C strain and the NP, P, M, F, HN, and L genes of a non-heat-resistant LaSota strain are interchanged one by one by using reverse genetic manipulation technology. Through studying changes of recombinant viruses in heat resistance after the gene interchange, it is found at last that HG gene is the key factor in determining heat resistance of NDV. This provides a theoretical basis and practical experience for the method for modification of heat resistance of NDV.
2) The complete genomic RNA of the non-heat-resistant NDV is linked into low copy number plasmids by using RT-PCR amplification technique and cloning, thus obtaining a transcription plasmid of the non-heat-resistant NDV.
3) An HN gene of a heat-resistant NDV is obtained through RT-PCR amplification, and is used to replace the HN gene in the transcription plasmid of the non-heat-resistant NDV through genetic cloning, thus obtaining a modified transcription plasmid.
4) A host cell is pre-infected with vaccinia virus which is capable of expressing T7 RNA polymerase, and is then co-transfected with the modified transcription plasmid and three helper plasmids (which express NP, P, and L proteins, respectively). The vaccinia virus expresses T7 RNA polymerase in the host cell. Then, the T7 RNA polymerase recognizes T7 promoter sequence in the transcription plasmid, initiates RNA replication, and terminates the replication at T7 terminator. The replicated RNA sequence is a genome of the non-heat-resistant NDV embedded with the HN gene of the heat-resistant NDV. The genomic RNA embedded with the HN gene of the heat-resistant NDV, together with the NP, P, and L proteins expressed by the three helper plasmids, forms a nucleoprotein complex, which initiates the first round of transcription of RNA virus as well as translation and synthesis of viral proteins. Each of components of the virus produces an infectious recombinant virus particle by self-assembly. The infectious recombinant virus particle comprises HN protein coming from the heat-resistant virus and other proteins coming from the non-heat-resistant virus. Since HN gene is the key heat-resistant factor of NDV, the recombinant virus based on the non-heat-resistant NDV and embedded with the HN gene of the heat-resistant NDV has a better heat resistance characteristic. The modification of heat resistance of the NDV is thus achieved.
5) Since different virus strains of NDV have very similar genomic structures and biological characteristics, many research approaches or technologies are applicable to different virus strains of NDV. For example, PCR technique of NDV can be used to identify all virus strains of NDV; method for reverse genetic manipulation of NDV virulent strains is also applicable to NDV low virulent strains; and the method of transforming the NDV low virulent LaSota strain into a virulent strain by modifying the cleavage site of the F protein of the low virulent LaSota strain is also applicable to modification of other NDV low virulent strains. Therefore, the method for modification of heat resistance of NDV provided by the present disclosure is applicable not only to LaSota strain but also to other NDV strains.

The present disclosure brings the following beneficial effects.
1) The method provided by the present disclosure is based on the transcription plasmid of the non-heat resistant NDV LaSota strain. The modified transcription plasmid is constructed by replacing the HN gene in the transcription plasmid of LaSota strain with the HN gene of heat-resistant NDV TS09-C strain, based on which a new recombinant virus is successfully obtained. Results of heat resistance testing experiments show that the heat resistance of the new recombinant virus is obviously stronger than that of the LaSota parent strain, which proves the capability of the method in modification of heat resistance of NDV. By using the method for modification of heat resistance of NDV provided by the present disclosure, a range of new recombinant heat-resistant NDV virus strains can be constructed and used in preparation of heat-resistant ND vaccines.
2) Compared with traditional ND vaccines, heat-resistant ND vaccines have the following advantages. a) Heat-resistant ND vaccines can be preserved at 4°C or even at room temperature, and therefore can save transport and preservation cost compared with commonly used vaccines which have to be cryopreserved. b) Heat-resistant ND vaccines are more stable in heat resistance, and hence have a longer shelf-life and can be used more effectively. c) Heat-resistant ND vaccines are easy to use, and can be administered via food, aerosol, eye-drop, nasal-drop, and so on.

### Brief Description of the Drawings

Fig. 1 schematically shows the construction of an NDV rT-HN strain after heat resistance modification;
Fig. 2 is a growth curve of the NDV rT-HN strain after heat resistance modification; and
Fig. 3 shows results of testing the heat resistance of the NDV rT-HN strain after heat resistance modification.

### Detailed Description of the Embodiments

The present disclosure will be explained in detail below with reference to the accompanying drawings and examples. However, it should be noted that the present disclosure is not restricted to the following examples.

### Example 1

### Construction of Transcription Plasmid of NDV Non-heat Resistant LaSota Strain

A transcription plasmid of NDV non-heat resistant LaSota strain contains a complete genomic sequence of LaSota strain, a promoter sequence of T7 RNA polymerase, a terminator sequence of T7 RNA polymerase, a ribozyme sequence of hepatitis delta virus, and a plasmid sequence of low copy number, etc., and was used mainly for transcription and expression and for accurate cleavage of the complete genomic RNA sequence of LaSota strain. A construction strategy of the transcription plasmid was as follows. First, the full-length genomic sequence was separated into 4 fragments from A to D and the 4 fragments were amplified separately. Of the 4 fragments, a T7 promoter sequence was added to the upstream of fragment A, and a ribozyme sequence of hepatitis delta virus and a T7 terminator sequence were added to the downstream of the fragment D. After the four fragments were obtained by performing amplification through conventional PCR, fusion PCR, and primer self-extension, the four fragments were linked into the low copy number plasmid in turn by In-fusion PCR cloning technique to obtain the transcription plasmid.

### 1. Extraction of RNA Virus and Reverse Transcription (RT) Reaction

Virus genomic RNA was extracted from allantoic fluid of a chick embryo infected with NDV LaSota strain according to the instruction of reagent kit. The total RNA was dissolved in 17 µl DEPC water. To the mixture was added 1 µl random primer. The mixture was then kept at 75° C for 5 min, and then immediately placed in a water bath. After that, a RT reaction solution containing 5 µl 5×RT Buffer, 1 µl dNTPs (10 mM), and 1µl M-MLV was added. After the resulting solution was kept at 42° C for 60 min, and then kept at 95° C for 5 min, the genomic cDNA of the virus was obtained, and was preserved at -20° C for PCR amplification.

### 2. Amplification and Clone of the Four Segments

Four pairs of PCR primers covering the full-length genome of the virus were designed and synthesized. To facilitate In-fusion clone, overlapping sections were provided among different fragments. PCR amplification of the full-length genomic DNA of the virus was performed by using virus cDNA as a template. A PCR system consisted of 10× Buffer, MgCl₂ (25 mM), dNTPs, upstream and downstream primers (10 µM), Taq enzyme, cDNA, and water. Different upstream and downstream primers were used for amplification of different segments. The conditions for PCR amplification were: 95° C for 5 min; 30 cycles: 94° C for 30 seconds, 55° C for 2 min, and 72° C for 5 min; and 72° C for 10 min. Each target band was detected through agarose gel electrophoresis. Specific positive band was purified and recovered through a DNA purification kit. The purified DNA was identified by endoglycosidase digestion. Identified PCR fragments were kept at -20°C for clone ligation.

### 3. Linking and Identification of the Transcription Plasmid

The four identified PCR segments were linked in turn into low copy number plasmid by In-fusion PCR cloning technique. The low copy number plasmid was digested with an appropriate endonuclease according to the instruction of the In-fusion PCR cloning reagent kit. Fragment A was first linked into the plasmid through cloning to obtain pBR-A. Most of the sequence of the pBR-A was obtained through PCR amplification. Both ends of the sequence of the pBR-A were connection sequences connecting fragment B. Linked cloning was performed between the pBR-A and fragment B to obtain pBR-AB. Fragments C and D were linked into the plasmid in similar ways. A plasmid pBR-ABCD, namely the transcription plasmid of LaSota strain, was thus obtained.

### 4. Construction of Helper Plasmids

PCR amplification was performed by using the transcription plasmid as a PCR template, to respectively obtain three genome segments encoding nucleoprotein, phosphoprotein, and large polymerase protein. The three segments were respectively cloned to eukaryotic expression vectors by enzyme digestion and ligation. To increase protein expression, Kozak sequence was inserted before an initiation codon. Plasmid was extracted from bacterial solution which was detected to be positive by PCR, and was then identified by enzyme digestion. The result showed that the constructed three helper plasmids pcDNA-NP, pcDNA-P and pcDNA-L were all correct. Sequence testing result was as expected, and the helper plasmids were successfully constructed.

### Example 2

### Construction of Transcription Plasmid of LaSota Strain with Modified HN Gene and Rescue of Virus

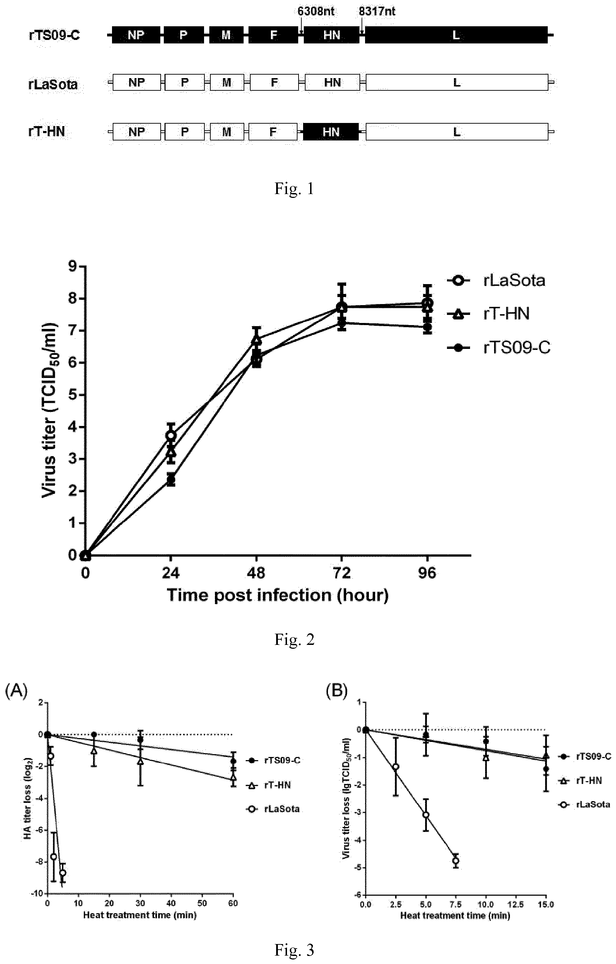

The HN gene in the transcription plasmid of LaSota strain was replaced with an HN gene of the heat-resistant NDV TS09-C strain, so as to obtain a transcription plasmid of LaSota strain with modified HN gene, as shown in Fig. 1. The modified transcription plasmid and the helper plasmids were used to co-transfect a host cell, to obtain a heat resistance-modified recombinant NDV.

### 1. PCR Amplification of HN Gene of the NDV TS09-C Strain

Virus genomic RNA was extracted from allantoic fluid of a chick embryo infected with NDV TS09-C strain according to the instruction of the reagent kit. The total RNA was dissolved in 17 µl DEPC water. HN gene was amplified by RT-PCR technique (sequences of two primers for the amplification respectively were: 5'-GTAGATGACCAAAGGGCGATATACGGGTAGAACGGT-3' and 5'-ACATTTTTTCTTAATCAAGTGACTACCG-3', where the underlined sequences represented fusion connection sequences). Each target band was detected through agarose gel electrophoresis. The specific positive band was purified and recovered through a DNA purification kit. The purified DNA was identified by endoglycosidase digestion. Identified PCR fragments were kept at -20° C.

### 2. Modification of HN Gene in the Transcription Plasmid of LaSota Strain

A pair of PCR primers was designed and synthesized for amplification of sequences of the transcription plasmid of LaSota strain other than the HN gene sequence thereof. A PCR system consisted of 10×Buffer, dNTPs, upstream and downstream primers (10 µM) (sequences of two primers for the amplification respectively were: 5'-ATTAAGAAAAAATGTAAGTGGCAATGAG -3' and 5'-CCTTTGGTCATCTACAACCGGTAG -3', where the underlined sequences represented fusion connection sequences), PFU Ultra II Fusion HS DNA polymerase, LaSota transcription plasmid, and water. The PCR amplification conditions were: 92° C for 2 min; 30 cycles: 92 °C for 10 seconds, 55° C for 20 seconds, and 68° C for 10 min; and 68° C for 10 min. Each target band was detected through agarose gel electrophoresis. The specific positive band was purified and recovered through a DNA purification kit. The purified DNA was identified by endoglycosidase digestion. The identified PCR fragments were kept at -20 °C. A PCR fragment (containing no HN gene) of the transcription plasmid of LaSota strain was thus obtained, and was named pLaSota-Del-HN.

The HN gene PCR fragments of TS09-C strain were linked to the transcription plasmid pLaSota-Del-HN through In-fusion cloning technique according the instruction of In-fusion PCR cloning reagent kit. The resulting product was transformed into competent bacteria. Following shaking of the bacterial, plasmid was extracted and identified by enzyme digestion and sequencing, to obtain a modified transcription plasmid of LaSota strain.

### 3. Rescue and Recovery of the Heat Resistance-Modified Virus and Identification Thereof

BHK-21 cells were seeded to a six-well plate. The cells grew to 80-90% confluence after sub-culturing for 1 day. The cell nutrient solution was substituted with a Dulbecco's modified Eagle medium (DMEM) culture medium with 2% new-born calf serum. The BHK-21 cells were infected with a vaccinia virus vTF7-3 at a multiplicity of infection (MOI) of 0.01 for 1 hour. After that, the modified transcription plasmid of LaSota strain and the three helper plasmids were used to co-transfect the BHK-21 cells by calcium phosphate method. The amounts of the plasmids used for co-transfection were respectively 2 µg, 0.5 µg, 0.5 µg, and 1 µg. After six hours from the co-transfection, the cell culture medium was substituted with a DMEM culture medium without new-born calf serum, followed by addition of tosyl-phenylalanine chloromethyl-ketone (TPCK) treated trypsin. After obvious pathological changes occurred to the cells, the cells were frozen and thawed twice. A supernatant was harvested. Vaccinia viruses were filtered out through a 0.22 µm filter membrane, and then subcultured for three continuous passages on the SPF chick embryos. The rescued recombinant virus was identified by HA potency testing and RT-PCR sequencing. Recombinant virus identified to be correct was a heat resistance-modified NDV virus, and was named rT-HN.

### Example 3

### Test of Biological Characteristics of the Heat Resistance-Modified NDV Virus

After modification of heat resistance of NDV LaSota strain, the new virus strain rT-HN was obtained. To determine whether the rT-HN strain had similar biological characteristics with the LaSota parent strain, biological characteristics of the recombinant virus rT-HN were tested.

### 1. Test of Cell Growth Characteristics of the NDV rT-HN Strain

BHK-21 cells were seeded to a 6-well plate, and grew into a compact monolayer in 24 hours. The cells were vaccinated with diluted rT-HN allantoic fluid. A control group of LaSota strain was provided. After 0, 24, 48, 72, and 96 hours from the vaccination, a cell culture supernatant was collected. The content of NDV in the supernatant was measured as follows. Ten-fold serial dilution of the supernatant was performed. The diluted supernatants were inoculated to BHK-21 cells that have grown into a compact monolayer in a 96-well plate, with dilution degrees ranging from 10⁻¹ to 10⁻⁸, an inoculation amount of 100 µl/well, and each dilution degree being repeated in three wells. After 1 hour of inoculation, the plate was washed with PBS twice. The culture medium was substituted with serum-free DMEM nutrient solutioncontaining TPCK-treated trypsin. The cells were cultivated, and pathological changes in the cells were observed and recorded. The content of the virus was calculated based on the number of wells in which pathological changes occurred, whereby a growth curve of the virus was obtained. As shown in Fig. 2, rT-HN strain had the highest titer (10^{7.1} TCID₅₀/ml) after 72 hours of infection, and had a similar growth curve with the LaSota parent strain.

### 2. Safety Test of the NDV rT-HN Strain in Chicken

Safety of the recombinant virus rT-HN in chicken was assessed by measuring a mean death time (MDT/MLD) and an intracerebral pathogenicity index (ICPC) of chick embryos. A control group of LaSota strain was provided. The MDT was determined as follows. A virus solution was 10-fold diluted using a physiological saline solution. The diluted solution was inoculated to SPF chick embryos at the age of 9 days with an inoculation amount of 0.1 ml/embryo. Eggs were candled twice each day and were observed for 7 consecutive days. Death time of chick embryos was recorded, and a MDT/MLD value was calculated. The ICPI was determined as follows. The 10-fold diluted virus solution was inoculated, with an inoculation amount of 0.05 ml/chick and in an intracerebral manner, to 10 SPF chicks that are 24 to 40 hours after hatching. The chicks were observed once a day and were graded. A normal chick was graded 0, a diseased chick was graded 1, and a dead chick was graded 2. The chicks were observed for 8 days, and an ICPC value was calculated. The result showed that the MDT value of the recombinant virus rT-HN was 121 hours, and the ICPI value thereof was 0.00. The MDT value of LaSota strain was 118 hours, and the ICPI value thereof was 0.00. Therefore, the recombinant virus rT-HN had a similar virulence with the LaSota parent strain.

### 3. Test of Heat-resistant Characteristic of the NDV rT-HN Strain

Allantoic fluid infected with rT-HN strain was placed into EP tubes, with an amount of 500 µl/tube. The tubes containing the virus solution were placed in a water bath at a temperature of 56° C. Virus solutions were respectively collected after 2.5 min, 5 min, 7.5 min, 10 min, 15 min, 30 min, and 60 min, and were used for measuring HA activity and virus titer. As shown in Fig. 3, compared with LaSota strain, the recombinant rT-HN strain had a distinctly improved infectivity heat resistance and HA heat resistance.

### Example 4

### Immunization Test of the Heat Resistance-Modified NDV rT-HN Strain

After determining that the heat resistance characteristic of the heat resistance-modified NDV rT-HN strain was significantly improved, the immunogenicity of the NDV rT-HN strain was measured. 40 one-day old SPF chicks were divided randomly into four groups, namely rT-HN group, rTS09-C group, rLaSota group, and PBS group, and were isolated. The chicks in each group were immunized with a corresponding virus sample by an amount of 0.1 ml and at a concentration of 10^{6.0}EID₅₀/ml through nose dropping/eye dropping. After 2 weeks from the immunization, serum was collected for HI potency test. In the meantime, the immunized chicks were challenged with NDV virulent F48E9 strain by an amount of 10^{4.0}EID₅₀/chick through nose dropping/eye dropping. After the challenge, the mobidity and mortality of the chicks were observed and recorded for 2 consecutive weeks, and immunization rate was calculated. Results of the immunization test were shown in Table 1. The protective rates in all the immunized groups of chicks were 100%, but the protective rate in chicks of the control group was 0%. HI antibody titer of the rT-HN group was slightly lower than that of the rLaSota strain, but was far higher than that of the rTS09-C group. It was thus determined that the NDV rT-HN strain had a similar immunogenicity with the parent LaSota strain, and thus could be used in preparation of ND heat-resistant live vaccines.

**Table 1. Immunization and Challenge Test of the Recombinant NDV**

| Virus Strains | Chick Number | Average Antibody Titer | Protective Rate |
|---|---|---|---|
| rT-HN | 10 | 6.50(0.58) | 10/10(100) |
| rTS09-C | 10 | 2.78(0.56) | 10/10(100) |
| rLaSota | 10 | 7.33(0.52) | 10/10(100) |
| PBS | 10 | 0.00(0.00) | 0/10(0) |

Nucleotide and Amino Acid Sequences
<110> Institute of Animal Husbandry and Veterinary Science, Hubei Academy of Agricultural Sciences
<120> Method for modification of heat resistance of Newcastle Disease Virus and use thereof
<160> 4
<210> 1
   <211> 1851 bp
   <212> DNA
   <213> artificial sequence
   <223> plasmid
<400> 1

atggaccgcg cagttagcca agttgcgcta gagaatgatg aaagagaggc aaagaataca 60
tggcgcttgg tattccggat cgcaatccta ctctcaacggtggtgacctt agccatctct 120
gcagccgccc ttgcatatag catggaggccagcacaccta gcgatcttgt aggcataccg 180
actgcgatct ctagagcaga ggaaaagatt acatctgcac tcggttccaa tcaagatgta 240
gtagatagga tatataagca ggtggccctc gaatctccac tggcattgct aaacaccgaa 300
tctacaatta tgaacgcaat aacgtctctc tcttatcaaa tcagtggggc cgcaagtagc 360
agcggatgtg gagcacccat tcatgatcca gattatattg gaggaatagg taaagaactt 420
attgtagatg atgctagcga cgtcacatca tactatccct ctgcgttcca agaacacctg 480
aactttatcc cggcgcctac tacaggatca ggttgcactc ggatgccctc atttgacatg 540
agcgctaccc actactgtta tactcacaat gtgatattat ctggctgcag agatcactcg 600
cactcacatc aatatttagc acttggtgtg cttcggacat ctgcaacagg gagggtattc 660
ttttccactc tgcgttccat caatctggat gacacccaaa atcggaagtc ttgcagtgtg 720
agtgcaaccc ccttgggttg tgatatgctg tgctctaaag tcacagagac tgaagaagag 780
gattataact cagctatccc cacgtcgatg gtacatggaa ggttagggtt cgacggccaa 840
taccacgaga aggacctaga tgtcacaaca ctattcgagg actgggtggc aaactaccca 900
ggagtaggag gcgggtcttt tattgacaac cgcgtatggt tcccagttta cggagggcta 960
aaacccaatt cgcccagtga caccgcacaa gaagggaaat atgtaatata caagcgatac 1020
aatgacacat gtccagatga gcaagattat cagattcaaa tggctaagtc ttcatataag 1080
cctgggcggt ttggagggaa acgcgtacag caggccgtct tatctatcaa agtgtcaaca 1140
tccttgggcg aggacccggt gctgactgta ccgcccaaca cagtaacact catgggggcc 1200
gaaggcagag ttctcacagt agggacatct catttccttt atcagcgagg gtcatcatac 1260
ttctcccctg ccctactata tcctatgata gtcagcaaca aaacagccac tcttcatagt 1320
ccttatacat tcaatgcctt cactcgacca ggtagtgtcc cttgccaggc ttcagcaaga 1380
tgccctaact catgtgttac cggagtctat actgatccat atcccttggt cttctatagg 1440
aaccacacct tgcgaggggt attcgggacg atgcttgatg ataaacaagc aagactcaac 1500
cctgtatctg cagtatttga cagcatatcc cgcagtcgca taacccgggt gagttcaagc 1560
agcaccaagg cagcatacac aacatcaaca tgttttaaag ttgtaaagac caataaaacc 1620
tattgtctca gcattgccga aatatccaat accctcttcg gggaattcag aatcgtccct 1680
ttactagttg agattctcaa ggatgatggg gttagagaag ccaggtctag ccggttgagt 1740
caactgcgag agggttggaa agatgacatt gtatcaccta tcttttgcga cgccaagaat 1800
caaactgaat accggcacga gctcgagtcc tacgctgcca gttggccata a 1851

## Claims

1. A method for modification of heat resistance of Newcastle disease virus, comprising steps of:
a. constructing a transcription plasmid of a non-heat-resistant Newcastle disease virus,
b. replacing the hemagglutinin-neuraminidase (HN) gene in the transcription plasmid of the non-heat-resistant Newcastle disease virus with the HN gene of a heat-resistant Newcastle disease virus, so as to obtain a modified transcription plasmid, and
c. co-transfecting a host cell with the modified transcription plasmid and three other helper plasmids, so as to obtain a heat resistance-modified recombinant Newcastle disease virus,
wherein the heat-resistant Newcastle disease virus is the TS09-C strain with the HN gene having the sequence of SEQ ID NO : 1; and
wherein the three helper plasmids express nucleoprotein, phosphoprotein, and large polymerase protein of the Newcastle disease virus, respectively.

2. The method for modification of heat resistance of Newcastle disease virus according to claim 1, wherein the transcription plasmid of the non-heat-resistant Newcastle disease virus expresses the whole genomic cDNA of the non-heat-resistant Newcastle disease virus.

3. The method for modification of heat resistance of Newcastle disease virus according to claim 2, wherein the non-heat-resistant Newcastle disease virus is the LaSota strain.

4. The method for modification of heat resistance of Newcastle disease virus according to claim 1, wherein:
primer sequences for amplification of the HN gene of the heat-resistant Newcastle disease virus are:
5'-GTAGATGACCAAAGGGCGATATACGGGTAGAACGGT-3' and
5'-ACATTTTTTCTTAATCAAGTGACTACCG-3', and
primer sequences for amplification of a fragment of the transcription plasmid of the non-heat-resistant Newcastle disease virus are:
5'-ATTAAGAAAAAATGTAAGTGGCAATGAG -3' and
5'- CCTTTGGTCATCTACAACCGGTAG -3', and
wherein the underlined sequence is a fusion connection sequence, through which two PCR products are fused and connected by in-fusion cloning technique, transformed into competent bacteria, and then plasmids are extracted by shaking the bacteria, identified by enzyme digestion and sequencing, thus obtaining the heat resistance-modified transcription plasmid.

5. A heat resistance-modified recombinant Newcastle disease virus which is the rT-HN strain deposited in China Center for Type Culture Collection under Accession Number CCTCC NO : V201529.

6. A heat-resistant live vaccine comprising the heat resistance-modified recombinant Newcastle disease virus according to claim 5 for use in preventing and/or controlling the Newcastle disease.

## Patentansprüche

1. Verfahren zum Modifizieren der Hitzebeständigkeit des Newcastle-Disease-Virus, umfassend die folgenden Schritte:
a. Herstellen eines Transkriptionsplasmids eines nicht hitzebeständigen Newcastle-Disease-Virus,
b. Ersetzen des Hämagglutinin -und Neuraminidase (HN) Gens in dem Transkriptionsplasmid des nicht hitzebeständigen Newcastle-Disease-Virus durch das HN-Gen eines hitzebeständigen Newcastle-Disease-Virus, um ein modifiziertes Transkriptionsplasmid zu erhalten, und
c. Cotransfektion einer Wirtszelle mit dem modifizierten Transkriptionsplasmid und drei anderen Helfer-Plasmiden, um einen Hitzebeständigkeits-modifiziertes, rekombinantes Newcastle-Disease-Virus zu erhalten,
wobei das hitzebeständige Newcastle-Disease-Virus der TS09-C Strang mit dem HN-Gen ist, das die Sequenz der SEQ ID Nr. : 1 hat, und
wobei die drei Helfer-Plasmiden jeweils Nukleoprotein, Phosphoprotein, und großes Polymeraseprotein des Newcastle-Disease-Virus exprimieren.

2. Verfahren zum Modifizieren der Hitzebeständigkeit des Newcastle-Disease-Virus nach Anspruch 1, wobei das Transkriptionsplasmid des nicht hitzebeständigen Newcastle-Disease-Virus die gesamte genomische cDNA des nicht hitzebeständigen Newcastle-Disease-Virus exprimiert.

3. Verfahren zum Modifizieren der Hitzebeständigkeit des Newcastle-Disease-Virus nach Anspruch 2, wobei das nicht-hitzebeständige Newcastle-Disease-Virus der LaSota-Stamm ist.

4. Verfahren zum Modifizieren der Hitzebeständigkeit des Newcastle-Disease-Virus nach Anspruch 1, wobei:
Primer-Sequenzen zur Verstärkung des HN-Gens des nicht-hitzebeständigen Newcastle-Disease-Virus sind:
5'-GTAGATGACCAAAGGGCGATATACGGGTAGAACGGT-3', und
5'-ACATTTTTTCTTAATCAAGTGACTACCG-3', und
Primer-Sequenzen zur Verstärkung eines Fragments des Transkriptionsplasmids des nicht-hitzebeständigen Newcastle-Disease-Virus sind:
5 '-ATTAAGAAAAAATGTAAGTGGCAATGAG-3' , und
5'- CCTTTGGTCATCTACAACCGGTAG -3', und
wobei die unterstrichene Sequenz eine Fusionsverbindungssequenz ist, durch die zwei PCR-Produkte fusioniert werden und durch die In-Fusion-Cloning Technik verbunden werden, in kompetente Bakterien umgewandelt werden und dann Plasmide durch Schütteln der Bakterien extrahiert werden, mittels Enzymdigestion und -sequenzierung identifiziert werden, wodurch das Hitzebeständigkeits-modifizierte Transkriptionsplasmid erhalten wird.

5. Hitzebeständigkeits-modifiziertes Newcastle-Disease-Virus, welches der rT-HN-Strang ist, der im China Center for Type Culture Collection unter der Zugangsnummer CCTCC- Nr.: V201529 hinterlegt ist.

6. Hitzebeständiger Lebendimpfstoff, aufweisend das Hitzebeständigkeits-modifizierte, rekombinante Newcastle-Disease-Virus nach Anspruch 5, zur Verwendung bei der Verhütung und/oder Kontrolle der Newcastle-Disease.

## Revendications

1. Procédé de modification de la résistance à la chaleur du virus de la maladie de Newcastle, comprenant les étapes consistant à :
a. construire un plasmide de transcription d'un virus de la maladie de Newcastle non résistant à la chaleur ;
b. remplacer le gène hémagglutinine-neuraminidase (HN) dans le plasmide de transcription du virus de la maladie de Newcastle non résistant à la chaleur avec le gène HN d'un virus de la maladie de Newcastle résistant à la chaleur, de manière à obtenir un plasmide de transcription modifié, et
c. co-transférer une cellule hôte avec le plasmide de transcription modifié et trois autres plasmides auxiliaires, de manière à obtenir un virus de la maladie de Newcastle modifié quant à la résistance à la chaleur recombinant,
dans lequel le virus de la maladie de Newcastle résistant à la chaleur est la souche TS09-C avec le gène HN présentant la séquence SEQ ID NO : 1, et
dans lequel les trois plasmides auxiliaires expriment respectivement une nucléoprotéine, une phosphoprotéine, et une grande protéine de polymérase du virus de la maladie de Newcastle.

2. Procédé de modification de la résistance à la chaleur du virus de la maladie de Newcastle selon la revendication 1, dans lequel le plasmide de transcription du virus de la maladie de Newcastle non résistant à la chaleur exprime l'ADN complémentaire génomique complet du virus de la maladie de Newcastle non résistant à la chaleur.

3. Procédé de modification de la résistance à la chaleur du virus de la maladie de Newcastle selon la revendication 2, dans lequel le virus de la maladie de Newcastle non résistant à la chaleur est la souche LaSota.

4. Procédé de modification de la résistance à la chaleur du virus de la maladie de Newcastle selon la revendication 1, dans lequel
les séquences d'amorce pour l'amplification du gène HN du virus de la maladie de Newcastle résistant à la chaleur sont :
5'-GTAGATGACCAAAGGGCGATATACGGGTAGAACGGT-3' et
5' -ACATTTTTTCTTAATCAAGTGACTACCG-3 ', et
les séquences d'amorce pour l'amplification d'un fragment du plasmide de transcription du virus de la maladie de Newcastle non résistant à la chaleur sont :
5' -ATTAAGAAAAAATGTAAGTGGCAATGAG-3' et
5' -CCTTTGGTCATCTACAACCGGTAG-3 ', et
dans lequel la séquence soulignée est une séquence de raccordement par fusion, par le biais de laquelle deux produits de réaction en chaîne par polymérase (PCR) sont fusionnés et raccordés par une technique de clonage par fusion, transformés en bactéries compétentes, puis des plasmides sont extraits en secouant les bactéries, identifiés par digestion enzymatique et séquençage, afin ainsi d'obtenir le plasmide de transcription modifié quant à la résistance à la chaleur.

5. Virus de la maladie de Newcastle modifié quant à la résistance à la chaleur recombinant, lequel est la souche rT-HN déposée auprès du China Center for Type Culture Collection sous le numéro d'entrée CCTCC NO : V201529.

6. Vaccin vivant résistant à la chaleur comprenant le virus de la maladie de Newcastle modifié quant à la résistance à la chaleur recombinant selon la revendication 5 à utiliser pour la prévention et/ou le contrôle de la maladie de Newcastle.
